# EUROPEAN PATENT SPECIFICATION

(11) **EP 2 858 702 B1**
(45) Date of publication and mention of the grant of the patent: **18.09.2019**
(21) Application number: 13737667.9
(22) Date of filing: 30.05.2013
(51) Int. Cl.: A61M 16/06

(54) **PATIENT INTERFACE FOR DELIVERING GAS TO A USER**
PATIENTENSCHNITTSTELLE ZUR ABGABE EINES GASES AN EINEN BENUTZER
INTERFACE PATIENT POUR ADMINISTRATION DE GAZ À UN UTILISATEUR

(30) Priority: 12.06.2012 US 201261658489 P
(43) Date of publication of application: 15.04.2015
(73) Proprietor: Koninklijke Philips N.V., 5656 AE Eindhoven (NL)
(72) Inventor: ZEIJLSTRA, Harmina Christina, 5656 AE Eindhoven (NL)
(74) Representative: Steffen, Thomas
(86) International application number: PCT/IB2013/054454
(87) International publication number: WO 2013/186657

(56) References cited:
- EP-A2- 2 301 614
- WO-A1-2008/040050
- WO-A1-2009/052560
- WO-A1-2011/048518
- WO-A1-2012/045127
- US-A1- 2009 032 026

## Description

### FIELD OF THE INVENTION

The present invention relates to a patient interface for use in pressure support systems that supply a flow of gas to the airway of a patient. In particular, the present invention relates to a patient interface device such as a facial mask.

### BACKGROUND OF THE INVENTION

Patient interfaces, such as masks in pressure support systems, are used for delivering gas to a user. Such gases like air, cleaned air, oxygen, or any modification thereof are submitted to the user (also referred to as patient) via the patient interface in a pressurized or unpressurized way.

For several chronic disorders and diseases, the usage of such a patient interface is necessary or at least advisable.

One non-limiting example of such a disease is obstructive sleep apnea or obstructive sleep apnea syndrome (OSA). OSA is usually caused by an obstruction of the upper airway. It is characterized by repetitive pauses in breathing during sleep and is usually associated with a reduction in blood oxygen saturation. These pauses in breathing, called apneas, typically last 20 to 40 seconds. The obstruction of the upper airway is usually caused by reduced muscle tonus of the body that occurs during sleep. The human airway is composed of walls of soft tissue which can collapse and thereby obstruct breathing during sleep. Tongue tissue moves towards the back of the throat during sleep and thereby blocks the air passages. OSA is therefore commonly accompanied with snoring. Different invasive and non-invasive treatments for OSA are known. One of the most powerful non-invasive treatments is the usage of Continuous Positive Airway Pressure (CPAP) or Bi-Positive Airway Pressure (BiPAP) in which a patient interface, e.g. a face mask, is attached to a tube and a machine that blows pressurized gas, preferably air, into the patient interface and through the airway of the patient in order to keep it open. Positive air pressure is thus provided to a patient through a hose connected to a patient interface or respiratory interface, such as a face mask, that is worn by the patient. The afore-mentioned long-term use of the patient interface is the result, since the wearing of the patient interface usually takes place during the sleeping time of the patient.

Examples for patient interfaces are:
- nasal masks, which fit over the nose and deliver gas through the nasal passages,
- oral masks, which fit over the mouth and deliver gas through the mouth,
- full face masks, which fit over both, the nose and the mouth, and deliver gas to both, and
- nasal pillows, which are regarded as masks as well within the scope of the present invention and which consist of small nasal inserts that deliver the gas directly to the nasal passages.

In order to guarantee a reliable operation of the device, the patient interface (mask) needs to closely fit on the patient's face to provide an air-tight seal at the mask-to-face interface. Usually, the patient interface is worn using a head gear with straps that go around the back of the patient's head. The patient interface or mask in practice usually comprises a soft cushion that is used as mask-to-patient interface, i.e. that contacts the face of the patient when the mask is worn, as well as it usually comprises a so-called mask shell building a rigid or semi-rigid holding structure for holding the cushion in place and for supplying mechanical stability to the patient interface (mask).

The cushion usually comprises one or more pads made of gel or silicon or any other soft material in order to increase the patient comfort and guarantee a soft feeling on the patient's face. The latter-mentioned mask shell usually also comprises a hose interface that is adapted for connecting the air supplying hose to the mask. Depending on the type of the mask, it may also comprise a mechanism with an additional cushion support on the forehead to balance the forces put by the mask around the airway entry features of the human face.

As such masks are usually worn over night and on a long-term basis, it is evident that e.g. cleaning of the mask is, especially for hygienic reasons, of great importance. It is therefore desirable that the mask may be disassembled into different parts in order to ease the cleaning of the mask. Apart from that, due to wear and tear, the cushions have to be replaced frequently. Independent of the reason for disassembling the mask and/or replacing the cushion, users in this case frequently have the problem of correctly reassembling the patient interface (mask). The mask cushion, for example, needs to be correctly and accurately positioned with respect to the mask shell holding the cushion. The lack of guidance in current products complicates the reassembling of the mask for the user. This does not only increase the risk of misalignment of the mask parts, but also, of course, decreases the user comfort.

Even more important, such a misalignment of the mask parts can result in leakage (e.g. air and/or pressure leakage) and/or cause excessive pressure points on the skin that may cause unpleasant and painful red marks.

EP 2 301 614 A2 proposes a respiratory mask assembly which makes use of simple alignment symbols that are provided on the frame and the cushion, which help the user to correctly align and orientate the frame with respect to the cushion. Such alignment symbols simplify the correct alignment between the different mask parts when reassembling the mask. However, the pretty simple alignment symbols proposed in EP 2 301 614 A2 have not received the best user feedback. Especially kids may find it hard to understand how to handle or to understand the alignment symbols or how to apply them in the correct way.

US 2009/0032026 A1, WO 2012/045127 A1 and WO 2008/040050 A1 all pertain to a respiratory mask having different types of alignment indicators. WO 2012/045127 A1 discloses alignment and/or orientation indicators for patient interfaces wherein the alignment indicators may form a continuous line over several components of the patient interface, the mask and portions of the headgear. The alignment, orientation, size and brand indicators may be molded and/or printed onto and/or into the mask.

### SUMMARY OF THE INVENTION

It is an object of the present invention to provide a patient interface for delivering gas to a user that allows to overcome the above-mentioned disadvantages. In particular, it is an object to provide a patient interface device for which the problem of misaligning the parts of the patient interface during assembly or re-assembly is efficiently overcome. Compared to state of the art patient interfaces, it shall be easier for the user to reassemble the patient interface after it has been disassembled, wherein the risk of misalignment of the parts of the patient interface is significantly reduced.

According to the present invention, this problem is solved by a patient interface for delivering gas to a user, with a first member and a second member that can be releasably connected to each other, wherein the first member comprises a first alignment marker and the second member comprises a second alignment marker, wherein the first and the second alignment markers cooperate to create a third alignment marker by overlapping each other when the first member and the second member are connected to each other, wherein the overlapped first and the second alignment markers together form the third alignment marker, wherein the third alignment marker is different from the first and second alignment marker, wherein the first alignment marker and/or the second alignment marker are at least partly visually transparent so that the third alignment marker is visually perceivable when the first and second alignment markers overlap each other, and wherein the third alignment marker is adapted to indicate proper alignment of the first and second member.

Preferred embodiments of the invention are defined in the dependent claims.

The invention overcomes the above-mentioned disadvantages by providing a first and second alignment marker which cooperate to create a third alignment marker when the first member and the second member of the patient interface are connected to each other. In other words, the proposed patient interface uses two alignment markers, one marker on each member of the patient interface (mask), which together form or results in a third marker when the first and the second alignment marker overlap each other, i.e. when the first and the second alignment marker are positioned on top of each other as soon as the first member of the mask is connected to the second member of the mask in correct and proper alignment.

In contrast to the simple alignment symbols proposed in EP 2 301 614 A2, where two identical alignment symbols are used which overlap in a proper alignment, the alignment technique used according to the present invention is much more intuitive for the user. The user may directly recognize if the two members of the patient interface are correctly aligned relative to each other when the third alignment marker visually appears. The third alignment marker therefore indicates a proper alignment of the first and the second member. In case of a misalignment, the first and the second alignment marker do not create the third alignment marker, i.e. no third alignment marker visually appears in this case. In still other words, the present invention makes use of an interaction between the first and the second alignment marker that together form a different (third) alignment marker when the first member and the second member of the patient interface are correctly aligned relative to each other.

In contrast to the simple visual alignment symbols used according to EP 2 301 614 A2, the alignment markers used according to the invention are more intuitive to handle, especially for kids. The first alignment marker could, for example, be realized as any coloured part of the first member of the mask, and the second alignment marker could be realized as a differently coloured corresponding part on the second member of the patient interface, so that the third alignment marker results from a colour mixture of the first and the second alignment marker. This resulting colour of the third alignment marker visually indicates that the two members of the patient interface are correctly aligned relative to each other. Especially kids may therefore easily see if they correctly reassembled the patient interface or not.

It is to be noted that the proposed technique may be applied for any type of patient interface, independent of a realization as a nasal mask, an oral mask, a full face mask, a total face mask or as nasal pillows. It shall be also noted that the described first and second members can be any parts of the patient interface, which can be releasably connected to each other. For example, the first and second member could refer to the mask cushion and the mask shell. However, the first and second member could also be the mask shell and the air supplying hose, which can usually be releasably-connected to each other as well. In most cases, a proper alignment of the two members of the mask is of importance, in particular to avoid unwanted air leaks and to guarantee an airtight seal of the mask.

According to an embodiment of the present invention, the first member comprises a first contact area and the second member comprises a second contact area, wherein the first contact area comprises the first alignment marker and the second contact area comprises the second alignment marker, and wherein the first and the second contact area at least partly overlap with each other when the first member and the second member are connected to each other.

In other words, the alignment markers are arranged on mating contact areas that overlap each other as soon as the first member and the second member are connected to each other. Due to this overlap of the two contact areas, the third alignment marker is automatically formed and/or appears in case the two members of the mask are properly aligned relative to each other. Thereto, the first contact area is preferably adapted to contact the second contact area when the first member and the second member are connected to each other. Any type of fixation means may be used to releasably connect the first and the second member with each other. In practice, the two members are preferably connected to each other by realizing a positive locking (form closure) or a non-positive connection (force closure) between the two parts of the mask.

According to a further embodiment, the first member comprises a cushion for contacting a face of the user, and the second member comprises a mask shell for holding the cushion. In other words, the first member may be realized as the cushion for contacting the face of the user and the second member may be realized as the mask shell for holding the cushion.

In this case, the second contact area may be formed as a recess in the mask shell and the first contact area may be a part of the cushion that may be placed in the recess to contact the cushion with the mask shell. The mask shell side of the cushion may be placed in the recess or groove in the mask shell to form a hermetic seal. As the above-explained alignment markers are in this case arranged on or within the recess of the mask shell and on the contact area at the mask shell side of the cushion (first contact area), the user him-/herself can visually determine a proper alignment between the cushion and the mask shell.

The above-described third alignment marker in this case visually indicates that the cushion is positioned correctly in the mask shell, i.e. that no gas leaks or misalignment occur at the interface between the cushion and the mask shell. The cushion may, for example, comprise a coloured ring forming the first contact area, which fits into the recess of the mask shell. The coloured ring guides the user in placing the cushion relative to the mask shell in the correct way.

Independent of the concrete design of the first and second alignment marker it is, according to an embodiment, preferred that the first alignment marker is different from the second alignment marker.

According to a further embodiment of the present invention, the first alignment marker has a first colour and the second alignment marker has a second colour different from the first colour, wherein the first and the second alignment marker cooperate to create the third alignment marker when the first member and the second member are connected to each other, and wherein the third alignment marker has a third colour different from the first and second colour.

In this embodiment, the colour of the cushion can, for example, be made in such a way that the colour becomes green when the cushion is placed correctly on the mask shell. The recess in the mask shell (the second contact area) may thereto, for example, have a yellow colour and the part of the cushion (first contact area) that fits into the recess of the mask shell may, for example, have a blue colour. As long as the recess, i.e. the one or more walls forming the recess of the mask shell, is at least partly transparent, the blue part of the cushion appears green when the cushion is properly attached to the mask shell. In other words, the third colour (e.g. green) representing the third alignment marker, is a colour mixture of the first and second colour (e.g. blue and yellow) representing the first and second alignment markers. Instead of using form markers or any specific signs or symbols, the first and the second alignment markers are in this case realized as coloured parts of the cushion and the mask shell. In the above-mentioned example, the user may directly identify a misalignment of the cushion if a blue part of the cushion may still be seen, i.e. if not all coloured parts of the cushion turned to green due to the overlap with the yellow coloured parts of the mask shell.

It is to be noted that the above-mentioned colours shall only be considered illustrative or exemplary and not restrictive. It is of course also possible to use differently coloured parts, e.g. yellow and red, green and red, or any other colour combination.

According to an embodiment of the present invention, it is preferred that the second colour is a complementary colour of the first colour. In this case, the third colour representing the third alignment marker results in a clear and well perceptible colour that may be easily identified by the user.

According to a further embodiment of the present invention, the first alignment marker covers the entire first contact area, and the second alignment marker covers the entire second contact area. In the above-mentioned example, this means that the whole part of the cushion that fits into the recess of the mask shell is entirely coloured in the first colour and the walls forming the mask shell and receiving the contacting part of the cushion are entirely coloured in the second colour. This even more simplifies the visual inspection of a correct alignment between these two parts for the user, since the user can inspect the alignment of the two parts from all spatial directions.

According to a further embodiment of the present invention, the third alignment marker is a visual icon, wherein the first alignment marker comprises a first part of the visual icon and wherein the second alignment marker comprises a second part of the visual icon.

In this embodiment, the first and the second alignment markers may not have to be coloured as explained above. However, the first and second alignment markers can also be coloured in this embodiment. In a conceivable practical realization of this embodiment, the first alignment marker could, for example, be a visual icon that has a form of a checkmark and the second alignment marker could have the form of a checkbox, so that the checkmark (first alignment marker) and the checkbox (second alignment marker) together form a checkmark within a checkbox if the two members of the patient interface (e.g. the cushion and the mask shell) are correctly aligned relative to each other. The combined checkmark within the checkbox in this case forms/represents the third alignment marker.

Alternatively, the third alignment marker could also be realized as a visual icon representing a picture of a smiley, wherein one part of the smiley represents the first alignment marker and the other part of the smiley represents the second alignment marker. In other words, the smiley only appears if the two members of the patient interface are correctly aligned relative to each other.

It is to be noted that the above-mentioned two examples are only illustrative and that also other visual icons can be realized for the third alignment marker, which may be generated by an overlap of the first and the second alignment markers.

All above-mentioned embodiments have in common that a first alignment marker on a first member cooperates with a second alignment marker on a second member of the patient interface and together form a new, different third alignment marker when the first member and the second member are connected to each other and properly aligned relative to each other, so that the third alignment marker indicates the proper alignment of the two members of the patient interface. It is clear that in all above-mentioned embodiments the first and/or the second alignment markers are at least partly visually transparent. Otherwise, the third alignment marker could not or at least not fully be visually perceivable when the fist and the second alignment markers overlap each other.

According to a further embodiment of the present invention, the first member comprises an additional fourth alignment marker and the second member comprises an additional fifth alignment marker which is identical to the fourth alignment marker, and wherein the fourth and the fifth alignment marker are adapted to overlap with each other and/or to gear into each other. In this embodiment, the fourth and the fifth alignment marker may be realized as coloured markers having a form of a dot or a line, or they may be realized as form markers that gear into each other.

In other words, coloured and/or former markers may be used in addition to the above-mentioned first and second alignment markers that cooperate with each other to form the third alignment marker. These additional markers may have various shapes and sizes and help to even more precisely identify a correct alignment of the two members of the patient interface.

### BRIEF DESCRIPTION OF THE DRAWINGS

These and other aspects of the invention will be apparent from and elucidated with reference to the embodiments described hereinafter. In the following drawings
Fig. 1 shows an example of a patient interface according to the present invention;
Figs. 2a, b schematically illustrate a first embodiment of the patient interface according to the present invention;
Figs. 3a, b schematically illustrate a second embodiment of the patient interface according to the present invention;
Figs. 4a, b schematically illustrate a third embodiment of the patient interface according to the present invention;
Figs. 5a, b schematically illustrate a fourth embodiment of the patient interface according to the present invention; and
Figs. 6a, b schematically illustrate a fifth embodiment of the patient interface according to the present invention.

### DETAILED DESCRIPTION OF THE INVENTION

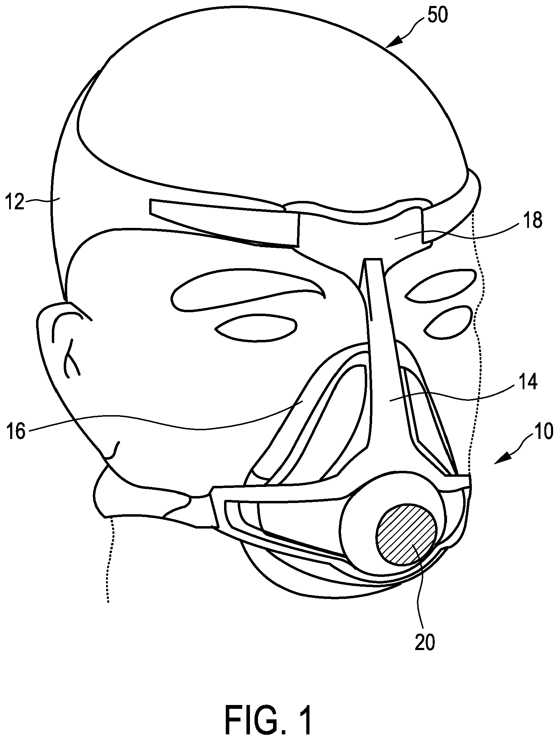

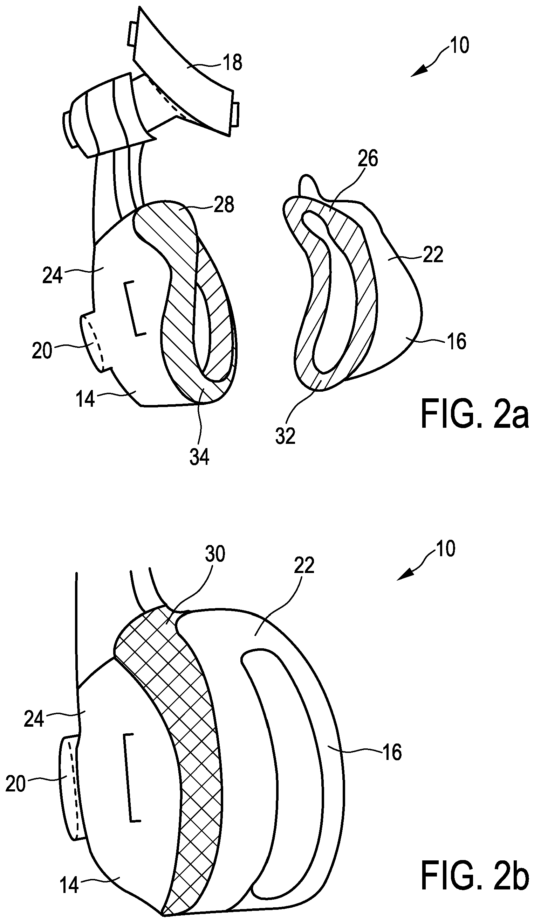

Fig. 1 shows an example of a CPAP mask according to the present invention. The main structural elements of the mask shown in Fig. 1 are generally known. The embodiments according to the present invention are shown in Figs. 2 to 6. However, Fig. 1 shall give an overview of the main structural elements comprised in such a mask.

The mask is in Fig. 1 in its entirety denoted with reference numeral 10. The mask 10, in the following also referred to as patient interface 10, is typically used in pressure support systems (CPAP systems) that supply a flow of gas to the airway of a patient 50. Such patient interfaces are well-known and are mostly worn on the head using a strap system 12 around the patient's head to hold the mask 10 in place around the airway entry features of the human face. The patient interface 10 typically comprises a rigid or semi-rigid mask shell 14 to which the head gear/strap system 12 is attached. The mask shell 14 is usually made of a rigid or semi-rigid material, such as e.g. plastic. However, also other materials are generally conceivable. The mask shell 14 serves as a holding frame for holding a flexible or soft cushion 16. The cushion 16 engages the patient's face when the mask/patient interface 10 is attached to the patient's face during use. It serves as mask-to-patient interface. These cushions 16 are usually made of silicon or comprise one or more gel pads in order to establish a soft contact on the patient's face. A further function of these cushions 16 is the sealing of the interior of the patient interface 10 to the exterior surrounding in order to prevent unwanted air leaks between the patient interface 10 and the patient's face when pressure is supplied to the patient's airway. The shape of the cushion 16 is thereto preferably adapted to the shape of the user's face.

The illustrated example refers to a so-called full-face mask 10, wherein the cushion 16 surrounds the nose and mouth of the user 50. These full-phase masks 10 often comprise an additional cushion support 18, also referred to as forehead support 18, which may be integrally connected to the mask shell 14, and which is arranged to engage the forehead of the patient 50. The additional cushion support/forehead support 18 mainly serves to balance the forces that the mask 10 exerts onto the face of the patient 50 and to mechanically stabilize the mask shell 14 as well as to serve for a correct and comfortable fit of the mask 10. A gas supplying hose (not shown) is usually connected to a connection interface 20 that is preferably attached to or integrated into the mask shell 14.

As already mentioned above, the example of the patient interface 10 shown in Fig. 1 refers to a so-called full-face mask. However, it is to be noted that this type of mask is herein included only for illustrative reasons in order to explain the core features of the present invention and make them apparent. However, the present invention is not restricted to any certain type of mask. The present invention may also be applied, for example, for nasal masks, oral masks, total face masks or nasal pillows.

The present invention, in particular, refers to a patient interface 10 for delivering gas to a user 50, wherein the alignment of the different sub-parts of the patient interface 10 is simplified in order to support the user/patient 50 to more easily and correctly assemble or reassemble the different sub-parts of the patient interface 10 with each other. In other words, the present invention shall support the user 50 to put the different sub-parts of the mask 10 together in a correct manner after he/she has disassembled the mask 10 for washing the different sub-parts or replacing one or all sub-parts with new sub-parts.

In practice, in particular the cushion 16 needs to be replaced or washed relatively often, since the cushion 16 commonly is a wear part that pollutes or even gets destroyed after a certain amount of time during the long-term use of the mask 10.

When having to reassemble the mask 10 or replace certain parts of the mask 10 users often have the problem of how to correctly align the different parts of the mask 10 relatively to each other. However, a correct assembly of the mask 10 is of utmost relevance, since otherwise air leaks may occur when the parts are not correctly attached to each other Apart from that an incorrect assembly may also lead to an incorrect fitting of the mask 10, which could lead to pressure marks on the patient's face that could cause unpleasant red marks on the face of the patient 50.

Fig. 2 shows a first embodiment of the patient interface 10 according to the present invention that overcomes the above-mentioned disadvantages of masks according to the prior art. The patient interface 10 comprises a first member 22 and a second member 24 that can be releasably connected to each other. The first member 22 in this example comprises the cushion 16 or is realized as the cushion 16, and the second member 24 comprises the mask shell 14 or is realized as the mask shell 14. However, it is to be noted that the terms "first and second members" may also refer to other parts of the mask 10 that can be releasably connected to each other, e.g. also to the air supplying hose (not shown) and the connection interface 20 (see Fig. 1), i.e. not necessarily as illustrated to the cushion 16 and the mask shell 14.

In the illustrated example, Fig. 2a illustrates the disassembled mask 10, i.e. in a condition in which the cushion 16 (the first member 22) is separated or unconnected from the mask shell 14 (second member 24). In contrast thereto, Fig. 2b illustrates the situation, in which the two members 22, 24 of the patient interface 10 (the cushion 16 and the mask shell 14) are connected to each other, i.e. in which the cushion 16 is inserted into the mask shell 14.

The first member 22 (in the illustrated example the cushion 16) comprises a first alignment marker 26 and the second member 24 (in the illustrated example the mask shell 14) comprises a second alignment marker 28. In contrast to simple alignment symbols known from the prior art, the first and the second alignment markers 26, 28 cooperate together to create a third alignment marker 30 when the first member 22 and the second member 24 are connected to each other. This third alignment marker 30 is adapted to indicate a proper alignment of the first and second member 22, 24. In the illustrated example, the third alignment marker 30 indicates whether the cushion 16 is in proper alignment with the mask shell 14, i.e. whether the cushion 16 is correctly attached to the mask shell 14.

The first alignment marker 26 is preferably different from the second alignment marker 28, and the third alignment marker 30 is different from the first and the second alignment marker 26, 28. In other words, the first and the second alignment marker 26, 28 are different alignment markers that together produce a third alignment marker 30, even different therefrom, when the first and the second alignment markers 26, 28 overlap each other.

In the concrete example illustrated in Figs. 2a, b the first and second alignment markers 26, 28 may be differently coloured parts of the first member 22 (the cushion 16) and the second member 24 (the mask shell 14), respectively. A part 32 of the cushion 16 may, for example, be coloured in blue, whereas a corresponding receiving member 34 of the mask shell 14 may, for example, be coloured in yellow. The part 32 of the cushion 16 may, for example, be released as a kind of ring 32 that surrounds the cushion 16 at the mask shell side, which ring/part 32 may be placed in the receiving member 34 of the mask shell 14, which receiving member is realized as a kind of recess or groove that receives the part/ring 32 of the cushion 16. As long as the receiving member 34 is at least partly transparent, the above-mentioned colourings (blue part 32 and yellow part 34) lead to a green appearance at the interface between the two members 22, 24 (between the cushion 16 and the mask shell 14). This green appearance or green-coloured part represents the third alignment marker 30.

A user is thus guided by the colours of the different parts 32, 34. The coloured ring 32 guides the user in placing the cushion 16 correctly into the coloured receiving member 34 of the mask shell 14. In the above-mentioned example, the user may directly see if he/she has inserted the cushion 16 in the correct way and if he/she has properly aligned the cushion 16 relative to the mask shell 14, when the cushion-to-mask shell interface appears green. As long as in the above-mentioned example any blue parts of the cushion ring 32 are still visible, this is an indicator for a wrong or incorrect alignment of the cushion 16 relative to the mask shell 14.

It shall be noted that the receiving member 34 does not necessarily need to have a shape of a recess or groove. Similarly, the above-mentioned part 32 of the cushion 16 does not necessarily need to be realized in the form of a cushion ring 32. Other shapes or technical designs are conceivable without departing from the present invention. In general, independent of their form, size and shape, these parts shall in the following be denoted generally as first contact area 32 and second contact area 34. Furthermore, it shall be noted that the above-mentioned colour examples are only given for illustrative reasons. However, also other colours can be generally used, e.g. a combination of yellow and green, green and red, blue and red, etc. In the above-given example, the first alignment marker 26 preferably has a different colour than the second alignment marker 28, and the resulting colour from the third alignment marker 30 is also different therefrom.

In summary, the first member 22 comprises a first contact area 32 and the second member 24 comprises a second contact area 34. The first contact area 32 comprises the first alignment marker 26, which is in this case realized as a coloured part of the first member 22 of the mask 10. The second contact area 34 comprises the second alignment marker 28, which is in this example realized as a coloured part of the mask shell 14 of the mask 10. The first and the second contact area 32, 34 at least partly overlap with each other when the first member 22 and the second member 24 are connected to each other. Due to this overlap the appearance of the coloured contact areas 32, 34 changes into a third colour representing the third alignment marker 30. In other words, the first and the second alignment markers 26, 28 cooperate with each other to create the third alignment marker 30, when the first member 22 and the second member 24 are connected to each other.

It is to be noted that according to the above-mentioned exemplary embodiment making use of coloured alignment markers 26, 28, the first and the second alignment markers 26, 28 may cover the entire first and second contact areas 32, 34. However, they may also only cover parts of these contact areas 32, 34. In order to receive a good visual contrast for the third alignment marker 30, it is preferred that the first and the second alignment markers 26, 28 are coloured in complementary colours. However, also other colours for the first and second alignment markers 26, 28 lead to an easily perceivable and distinguishable third colour mixture for the third alignment marker 30.

Instead of using coloured alignment markers 26, 28 the first and second alignment marker 26, 28 may also be realized by parts having different refractive indices, so that a different appearance occurs for the third alignment marker 30 as soon as the first and second alignment marker 26, 28 overlap each other. This could, for example, also be realized by making use of polarizing filters.

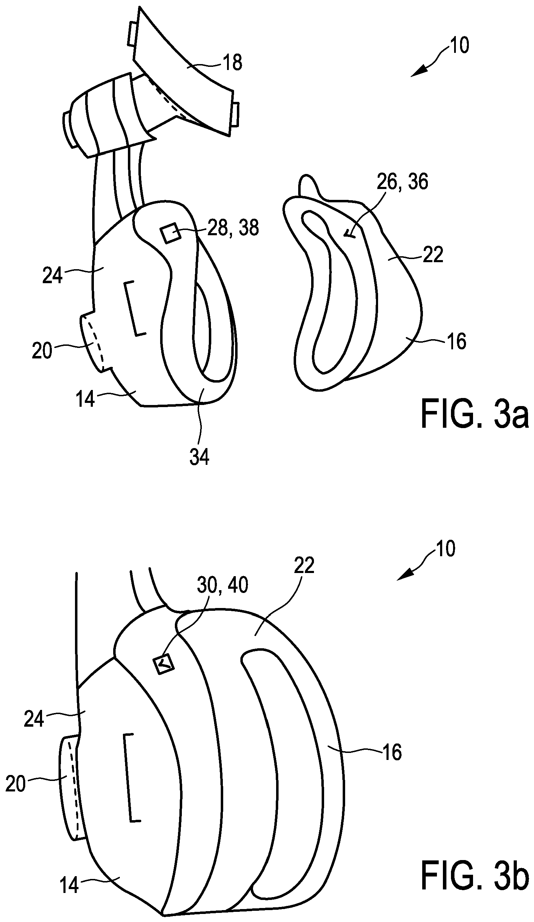

Further embodiments of the present invention are schematically illustrated in Figs. 3 to 6. Figs. 3 to 6 refer to embodiments, wherein the first and second alignment markers 26, 28 are realized as icons, in contrast to the coloured alignment markers 26, 28 explained with reference to Fig. 1. The first alignment marker therein comprises a first part 36 of a visual icon 40, and the second alignment marker 28 comprises a second part 38 of the visual icon 40. In other words, both alignment markers 26, 28 consist of or comprise two different parts 36, 38 of an icon 40, and create the icon 40 when being overlapped.

In the example shown in Figs. 3a, b the first alignment marker 26 is realized as a checkmark 36 and the second alignment marker 28 is realized as a checkbox 38, so that the resulting third alignment marker 30 appears as a checkmark within a checkbox (see reference numeral 40). The user may therefore see if the first member 22 is correctly aligned relative to the second member 24 when the checkmark 36 correctly appears within the checkbox 38.

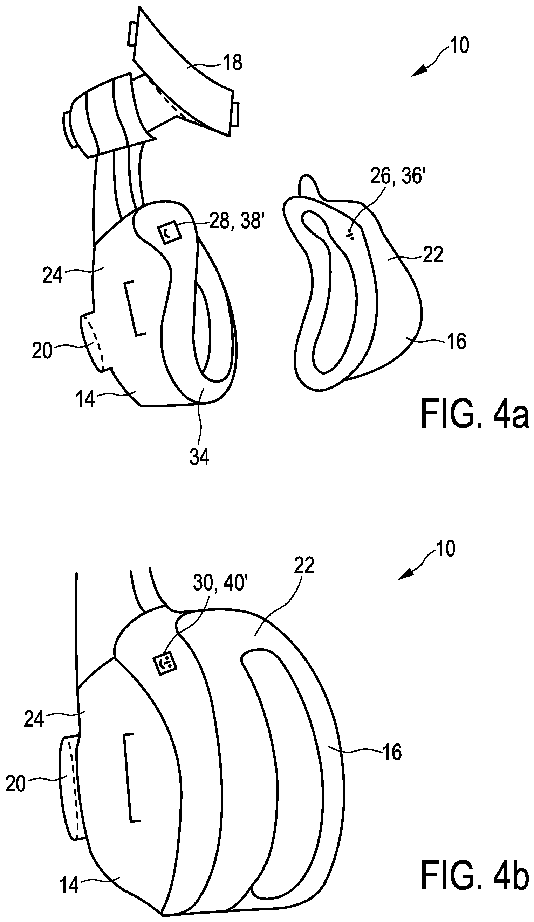

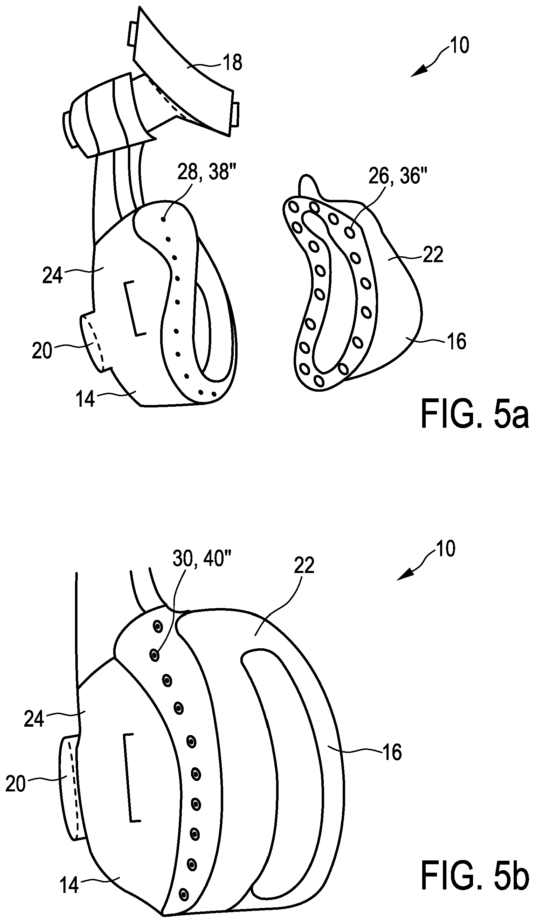

Alternatively, as shown in Figs. 4a, b the third alignment marker 30 may be realized as a visual icon 40' that represents a smiley. The first alignment marker 26 in this case comprises a first part 36 of the smiley 40', and the second alignment marker 28 comprises a second part 38' of the smiley 40'. Similarly, it is also conceivable to use circles 36" and dots 38" fitting in the circles 36" to create a third example of the third alignment marker 30. In this case the resulting visual icon 40" representing the third alignment marker comprises several circles 36" which include dots 38" in their center (see Figs. 5a, b).

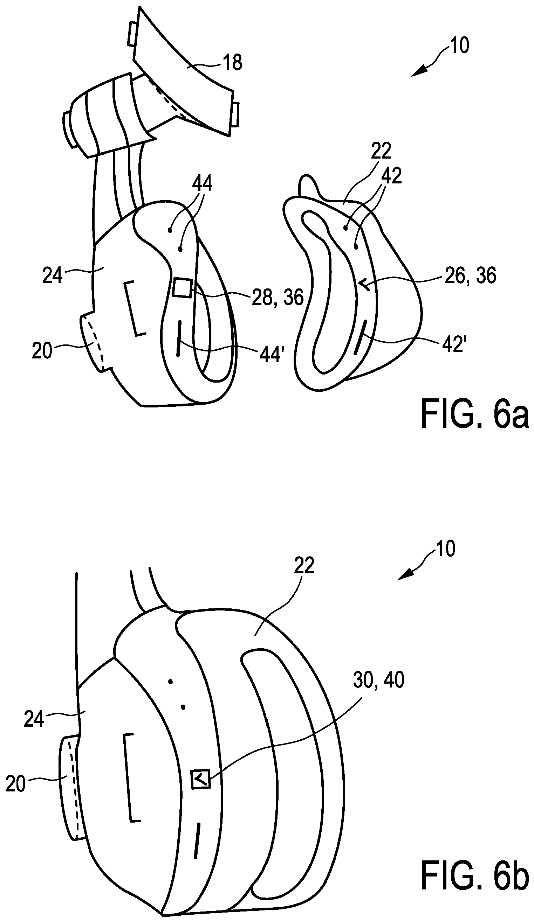

As shown in Figs. 6a, b additional fourth alignment markers 42, 42' and fifth alignment markers 44, 44' can be used to support the user 50 to correctly align the first part 22 relative to the second part 24 of the mask 10. The first member 22 may comprise the additional forth alignment markers 42, 42' and the second member 24 may comprise the additional fifth alignment markers 44, 44' which are identical to the fourth alignment markers 42, 42'. The fourth and the fifth alignment markers 42, 42', 44, 44' are adapted to overlap with each other and/or to gear into each other. The forth and the fifth alignment markers can be realized as coloured markers having a form of a dot, a line, a pyramid, a circle or any other shape. However, the fourth and the fifth alignment markers can also be realized as form markers, i.e. being realized by one or more protrusions that fit or gear into corresponding recesses on the other member. Independent of their shape, these markers 42, 42', 44, 44' can also be realized as coloured markers. Laser markings could also be applied as visual or form markings.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Patient interface for delivering gas to a user (50), with a first member (22) and a second member (24) that can be releasably connected to each other, wherein the first member (22) comprises a first alignment marker (26) and the second member (24) comprises a second alignment marker (28), wherein the first and the second alignment markers (26, 28) cooperate to create a third alignment marker (30) by overlapping each other when the first member (22) and the second member (24) are connected to each other, wherein the overlapped first and the second alignment markers (26, 28) together form the third alignment marker (30), wherein the third alignment marker (30) is different from the first and second alignment marker (26, 28), wherein the first alignment marker (26) and/or the second alignment marker (28) are at least partly visually transparent so that the third alignment marker (30) is visually perceivable when the first and second alignment markers (26, 28) overlap each other, and wherein the third alignment marker (30) is adapted to indicate proper alignment of the first and second member (22, 24).

2. Patient interface according to claim 1, wherein the first member (22) comprises a first contact area (32) and the second member (24) comprises a second contact area (34), wherein the first contact area (32) comprises the first alignment marker (26) and the second contact area (34) comprises the second alignment marker (28), and wherein the first and the second contact area (32, 34) at least partly overlap with each other when the first member (22) and the second member (24) are connected to each other.

3. Patient interface according to claim 2, wherein the first contact area (32) is adapted to contact the second contact area (34) when the first member (22) and the second member (24) are connected to each other.

4. Patient interface according to claim 1, wherein the first member (22) comprises a cushion (16) for contacting a face of the user (50), and wherein the second member (24) comprises a mask shell (14) for holding the cushion (16).

5. Patient interface according to claims 2 and 4, wherein the second contact area (34) is formed as a recess in the mask shell (14) and the first contact area (32) is a part of the cushion (16) that may be placed in the recess to connect the cushion (16) with the mask shell (14).

6. Patient interface according to claim 1, wherein the first alignment marker (26) is different from the second alignment marker (28).

7. Patient interface according to claim 1, wherein the first alignment marker (26) has a first colour and the second alignment marker (28) has a second colour different from the first colour, wherein the first and second alignment markers (26, 28) cooperate to create the third alignment marker (30) when the first member (22) and the second member (24) are connected to each other, and wherein the third alignment marker (30) has a third colour different from the first and second colour.

8. Patient interface according to claim 7, wherein the third colour is a colour mixture of the first and second colour.

9. Patient interface according to claim 7, wherein the second colour is a complementary colour of the first colour.

10. Patient interface according to claim 2, wherein the first alignment marker (26) covers the entire first contact area (32), and wherein the second alignment marker (28) covers the entire second contact area (34).

11. Patient interface according to claim 1, wherein the third alignment marker (30) is a visual icon (40, 40', 40"), wherein the first alignment marker (26) comprises a first part (36, 36', 36") of the visual icon (40, 40', 40"), and wherein the second alignment marker (28) comprises a second part (38, 38', 38") of the visual icon (40, 40', 40").

12. Patient interface according to claim 1, wherein the first member (22) comprises an additional fourth alignment marker (42, 42') and the second member (24) comprises an additional fifth alignment marker (44, 44') which is identical to the fourth alignment marker (42, 42'), and wherein the fourth and the fifth alignment marker (42, 42', 44, 44') are adapted to overlap with each other and/or to gear into each other.

## Patentansprüche

1. Patientenübergangsstelle für die Zuleitung von Gas zu einem Benutzer (50) mit einem ersten Element (22) und einem zweiten Element (24), die lösbar miteinander verbunden sein können, wobei das erste Element (22) einen ersten Ausrichtungsmarker (26) umfasst und das zweite Element (24) einen zweiten Ausrichtungsmarker (28) umfasst, wobei der erste und der zweite Ausrichtungsmarker (26, 28) kooperieren, um einen dritten Ausrichtungsmarker (30) zu erzeugen, indem sie einander überlappen, wenn das erste Element (22) und das zweite Element (24) miteinander verbunden sind, wobei der überlappte erste und zweite Ausrichtungsmarker (26, 28) gemeinsam den dritten Ausrichtungsmarker (30) bilden, wobei der dritte Ausrichtungsmarker (30) von dem ersten und zweiten Ausrichtungsmarker (26, 28) verschieden ist, wobei der erste Ausrichtungsmarker (26) und/oder der zweite Ausrichtungsmarker (28) mindestens teilweise visuell transparent sind, sodass der dritte Ausrichtungsmarker (30) visuell wahrnehmbar ist, wenn der erste und zweite Ausrichtungsmarker (26, 28) einander überlappen, und wobei der dritte Ausrichtungsmarker (30) angepasst ist, um eine korrekte Ausrichtung des ersten und zweiten Elements (22, 24) anzuzeigen.

2. Patientenübergangsstelle nach Anspruch 1, wobei das erste Element (22) eine erste Kontaktfläche (32) umfasst und das zweite Element (24) eine zweite Kontaktfläche (34) umfasst, wobei die erste Kontaktfläche (32) den ersten Ausrichtungsmarker (26) umfasst und die zweite Kontaktfläche (34) den zweiten Ausrichtungsmarker (28) umfasst und wobei die erste und die zweite Kontaktfläche (32, 34) einander mindestens teilweise überlappen, wenn das erste Element (22) und das zweite Element (24) miteinander verbunden sind.

3. Patientenübergangsstelle nach Anspruch 2, wobei die erste Kontaktfläche (32) angepasst ist, um mit der zweiten Kontaktfläche (34) in Kontakt zu treten, wenn das erste Element (22) und das zweite Element (24) miteinander verbunden sind.

4. Patientenübergangsstelle nach Anspruch 1, wobei das erste Element (22) eine Polsterung (16) zum In-Kontakt-Treten mit einem Gesicht des Benutzers (50) umfasst und wobei das zweite Element (24) eine Maskenschale (14) zum Halten der Polsterung (16) umfasst.

5. Patientenübergangsstelle nach den Ansprüche 2 und 4, wobei die zweite Kontaktfläche (34) als Aussparung in der Maskenschale (14) gebildet ist und die erste Kontaktfläche (32) Teil der Polsterung (16) ist, die in der Vertiefung platziert werden kann, um die Polsterung (16) mit der Maskenschale (14) zu verbinden.

6. Patientenübergangsstelle nach Anspruch 1, wobei der erste Ausrichtungsmarker (26) von dem zweiten Ausrichtungsmarker (28) verschieden ist.

7. Patientenübergangsstelle nach Anspruch 1, wobei der erste Ausrichtungsmarker (26) eine erste Farbe aufweist und der zweite Ausrichtungsmarker (28) eine zweite Farbe aufweist, die von der ersten Farbe verschieden ist, wobei der erste und zweite Ausrichtungsmarker (26, 28) kooperieren, um einen dritten Ausrichtungsmarker (30) zu erzeugen, wenn das erste Element (22) und das zweite Element (24) miteinander verbunden sind, und wobei der dritte Ausrichtungsmarker (30) eine dritte Farbe aufweist, die von der ersten und zweiten Farbe verschieden ist.

8. Patientenübergangsstelle nach Anspruch 7, wobei die dritte Farbe eine Farbmischung aus der ersten und zweiten Farbe ist.

9. Patientenübergangsstelle nach Anspruch 7, wobei die zweite Farbe eine Komplementärfarbe zu der ersten Farbe ist.

10. Patientenübergangsstelle nach Anspruch 2, wobei der erste Ausrichtungsmarker (26) die gesamte erste Kontaktfläche (32) bedeckt und wobei der zweite Ausrichtungsmarker (28) die gesamte zweite Kontaktfläche (34) bedeckt.

11. Patientenübergangsstelle nach Anspruch 1, wobei der dritte Ausrichtungsmarker (30) ein visuelles Icon (40, 40', 40") ist, wobei der erste Ausrichtungsmarker (26) einen ersten Teil (36, 36', 36") des visuellen Icons (40, 40', 40") umfasst und wobei der zweite Ausrichtungsmarker (28) einen zweiten Teil (38, 38', 38") des visuellen Icons (40, 40', 40") umfasst.

12. Patientenübergangsstelle nach Anspruch 1, wobei das erste Element (22) einen zusätzlichen vierten Ausrichtungsmarker (42, 42') umfasst und das zweite Element (24) einen zusätzlichen fünften Ausrichtungsmarker (44, 44') umfasst, der mit dem vierten Ausrichtungsmarker (42, 42') identisch ist, und wobei der vierte und der fünfte Ausrichtungsmarker (42, 42', 44, 44') angepasst sind, um einander zu überlappen und/oder ineinander einzugreifen.

## Revendications

1. Interface patient pour distribuer du gaz à un utilisateur (50), avec un premier élément (22) et un deuxième élément (24) qui peuvent être reliés de manière détachable l'un à l'autre, dans laquelle le premier élément (22) comprend un premier repère d'alignement (26) et le deuxième élément (24) comprend un deuxième repère d'alignement (28), dans laquelle le premier et le deuxième repère d'alignement (26, 28) coopèrent pour créer un troisième repère d'alignement (30) en se chevauchant quand le premier élément (22) et le deuxième élément (24) sont reliés l'un à l'autre, dans laquelle les premier et deuxième repères d'alignement (26, 28) se chevauchant forment ensemble le troisième repère d'alignement (30), dans laquelle le troisième repère d'alignement (30) est différent des premier et deuxième repères d'alignement (26, 28), dans laquelle le premier repère d'alignement (26) et/ou le deuxième repère d'alignement (28) sont au moins partiellement visuellement transparents de telle sorte que le troisième repère d'alignement (30) peut être visuellement perçu quand les premier et deuxième repères d'alignement (26, 28) se chevauchent, et dans lequel le troisième repère d'alignement (30) est adapté pour indiquer un alignement correct des premier et deuxième éléments (22, 24).

2. Interface patient selon la revendication 1, dans laquelle le premier élément (22) comprend une première zone de contact (32) et le deuxième élément (24) comprend une deuxième zone de contact (34), dans laquelle la première zone de contact (32) comprend le premier repère d'alignement (26) et la deuxième zone de contact (34) comprend le deuxième repère d'alignement (28), et dans laquelle les première et deuxième zones de contact (32, 34) se chevauchent au moins partiellement quand le premier élément (22) et le deuxième élément (24) sont reliés l'un à l'autre.

3. Interface patient selon la revendication 2, dans laquelle la première zone de contact (32) est adaptée pour entrer en contact avec la deuxième zone de contact (34) quand le premier élément (22) et le deuxième élément (24) sont reliés l'un à l'autre.

4. Interface patient selon la revendication 1, dans laquelle le premier élément (22) comprend un coussinet (16) pour être en contact avec un visage de l'utilisateur (50), et dans laquelle le deuxième élément (24) comprend une coque de masque (14) pour maintenir le coussinet (16).

5. Interface patient selon les revendications 2 et 4, dans laquelle la deuxième zone de contact (34) est formée en tant qu'évidement dans la coque de masque (14) et la première zone de contact (32) est une partie du coussinet (16) qui peut être placée dans l'évidement pour relier le coussinet (16) à la coque de masque (14).

6. Interface patient selon la revendication 1, dans laquelle le premier repère d'alignement (26) est différent du deuxième repère d'alignement (28).

7. Interface patient selon la revendication 1, dans laquelle le premier repère d'alignement (26) a une première couleur et le deuxième repère d'alignement (28) a une deuxième couleur différente de la première couleur, dans laquelle les premier et deuxième repères d'alignement (26, 28) coopèrent pour créer le troisième repère d'alignement (30) quand le premier élément (22) et le deuxième élément (24) sont reliés l'un à l'autre, et dans laquelle le troisième repère d'alignement (30) a une troisième couleur différente des première et deuxième couleurs.

8. Interface patient selon la revendication 7, dans laquelle la troisième couleur est un mélange de couleurs des première et deuxième couleurs.

9. Interface patient selon la revendication 7, dans laquelle la deuxième couleur est une couleur complémentaire de la première couleur.

10. Interface patient selon la revendication 2, dans laquelle le premier repère d'alignement (26) recouvre toute la première zone de contact (32), et dans laquelle le deuxième repère d'alignement (28) recouvre toute la deuxième zone de contact (34).

11. Interface patient selon la revendication 1, dans laquelle le troisième repère d'alignement (30) est une icône visuelle (40, 40', 40"), dans laquelle le premier repère d'alignement (26) comprend une première partie (36, 36', 36") de l'icône visuelle (40, 40', 40"), et dans laquelle le deuxième repère d'alignement (28) comprend une deuxième partie (38, 38', 38") de l'icône visuelle (40, 40', 40").

12. Interface patient selon la revendication 1, dans laquelle le premier élément (22) comprend un quatrième repère d'alignement supplémentaire (42, 42') et le deuxième élément (24) comprend un cinquième repère d'alignement supplémentaire (44, 44') qui est identique au quatrième repère d'alignement supplémentaire (42, 42'), et dans laquelle le quatrième et le cinquième repère d'alignement (42, 42', 44, 44') sont adaptés pour se chevaucher et/ou pour s'engrener mutuellement.
